# EUROPEAN PATENT SPECIFICATION

(11) **EP 4 319 591 B1**
(45) Date of publication and mention of the grant of the patent: **27.05.2026**
(21) Application number: 21936170.6
(22) Date of filing: 16.06.2021
(51) Int. Cl.: A61L 31/04, A41D 19/00, A61K 8/02, A61K 8/36, A61K 8/9789, A61K 8/9794, A61Q 19/00, A61Q 19/08, A61Q 19/02, C08J 7/04, A61M 35/00, A61K 36/258, A61K 36/77, A61K 36/886, A61L 31/06, A61L 31/14, A61L 31/16

(54) **AN ELASTOMERIC ARTICLE**
ELASTOMERISCHER ARTIKEL
ARTICLE ÉLASTOMÈRE

(30) Priority: 09.04.2021 MY PI2021001968
(43) Date of publication of application: 14.02.2024
(73) Proprietor: Matang Manufacturing Sdn Bhd, Kuala Lumpur, 60000 (MY)
(72) Inventor: LEONG, Nicholas, Mum Fook, Kuala Lumpur, 60000 (MY); LEONG, Ronald, Low Pew, Kuala Lumpur, 60000 (MY)
(74) Representative: Lavoix
(86) International application number: PCT/MY2021/050047
(87) International publication number: WO 2022/216148

(56) References cited:
- WO-A1-2004/037305
- WO-A1-2017/191629
- WO-A1-2018/119491
- CN-A- 111 450 023
- US-A1- 2004 091 557
- US-A1- 2017 071 848
- US-B2- 6 589 544

## Description

### Field of the Invention

The present invention relates to an elastomeric article and a method of making thereof. More particularly, the present invention relates to improved elastomeric articles with emollient and rejuvenative characteristics.

### Background of the Invention

An elastomer is a polymer characterised by the properties of elasticity and resilience. An elastomer is available in both natural and synthetic types with varying mechanical properties. It is often used in the formation of flexible articles, such as gloves, liners, finger cots, sheaths, cervical caps, dental dams, and condoms. The flexibility and impermeability of the articles provide a good barrier and protection to the user.

Gloves are elastomeric articles designed to protect the wearer against chemical, infectious, or other hazardous substances. It is widely and frequently used in laboratory and hospitals when dealing with hazardous materials or high-risk patients. On the other hand, excess dryness and irritation of the skin are common problems when wearing the gloves for extended periods.

Hence, elastomeric articles or gloves with various inner treatments have been devised to overcome the undesired effects. For example, WO 2018/119491 A1 teaches elastomeric articles that can deliver skincare properties effectively to a person wearing or coming into contact with the articles. The articles comprise an elastomeric film and a plant stem cell material. The plant stem cell material contains skincare properties and can be in the form of a bilayer membrane-encapsulated plant stem cell material or present as a layer or coating on a surface of the elastomeric film.

US 2004/0091504 A1 provides flexible elastomer articles with improved moisturising and donning properties. A botanical extract having a polysaccharide is incorporated into an elastomer emulsion, solution and/or plastisol to enhance the physical and therapeutic properties of articles made from these materials. In another aspect, the flexible elastomer articles are coated with a non-*Aloe vera* mucinous botanical or laboratory produced polysaccharide which is fortified by additives known to protect, restore and moisturise mammalian skin or mucosa and to enhance ease of application or donnability of the article.

US 6,692,756 B2 discloses a moisturising and therapeutic glove which includes a thin layer of *Aloe Vera* coated evenly and uniformly on an inside surface of the glove. *Aloe Vera* is attached to the surface through a dehydration process achieved with a controlled drying method. Hence, the glove is a single layer glove with a dehydrated coating of *Aloe vera.*

US 6,589,544 B2 teaches a method of manufacturing a thin-walled elastomeric article with incorporated *Aloe vera.* The method comprises dipping an article-shaped form into a latex composition that has been mixed with the extract of *Aloe vera.* The form is then processed and cured so that the extract is present within the article as one or combination of a polymer, co-polymer and filler of the article.

While elastomer articles with different inner treatments or skincare properties are available in a wide range of forms, degradation of the skincare properties during the process of manufacturing or storage has barely been addressed.

In view of the above, there is a need for an elastomeric article and a method of making the article, which enable the preservation of the efficacy and stability of the skincare properties.

### Summary of the Invention

An object of the present invention is to provide an elastomeric article with therapeutic properties.

Another object of the present invention is to provide an elastomeric article with an extract of soapnuts to preserve the beneficial properties of therapeutic agents.

A further object of the present invention is to provide a low-cost method of making the aforesaid articles.

### Brief Description of Drawings

Fig. 1 is a front perspective view showing an elastomeric article, such as a glove, constructed in accordance with an embodiment of the present invention.
Fig. 2 is sectional view taken along the line A-A of Fig. 1, showing an elastomeric article comprises an intermediate coating and an inner coating in accordance with an embodiment of the present invention.
Fig. 3 is a sectional view taken along the line A-A of Fig. 1, showing an elastomeric article comprises an inner coating in accordance with an embodiment of the present invention.
Fig. 4 is a schematic flow diagram showing a method of making an elastomeric article in accordance with an embodiment of the present invention.

### Detailed Description of Embodiments

The present invention relates to elastomeric articles (10) and methods of making the articles (10). More particularly, the present invention relates to an elastomeric article (10) with a therapeutic agent and an extract of soapnuts.

As used herein, the term "therapeutic agent" refers to any therapeutic components having an advantageous or restorative effect on the body, particularly on the skin. The therapeutic agent may comprise but not limited to one or more of anti-inflammatory, anti-microbial, anti-aging, antioxidant, wound healing, and cosmetic properties. The therapeutic agent may include a composition or a formulation with mosturising, emollient, and rejuvenative effect.

Preferably, the therapeutic agent comprises a plant-based extract. The plant-based extract may be in a form of gel, powder, or solution.

More preferably, the plant-based extract is an extract of a plant selected from *Aloe vera,* ginseng, lotus, or any combination thereof.

Further preferably, the plant-based extract comprises extracts of *Aloe vera* and ginseng. *Aloe vera* is known for its moisturising, hydrating, and soothing properties, and the plant is generally tolerable to irritable or allergic skin. Additionally, ginseng contains natural substances that promote whitening and is effective in preventing signs of skin aging. The substances of ginseng are heat-resistant, and hence the efficacy of the substances will not be compromised under the excessive temperature in the process of forming the article (10).

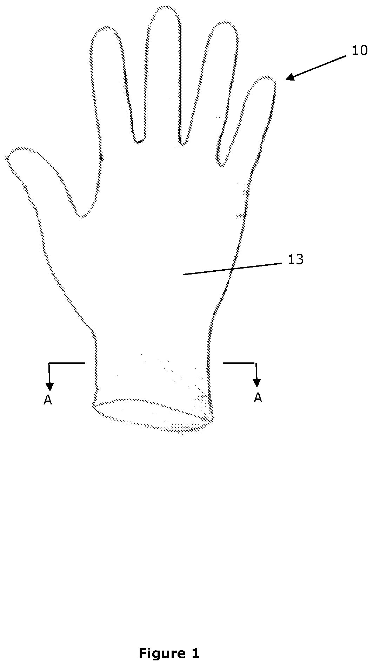

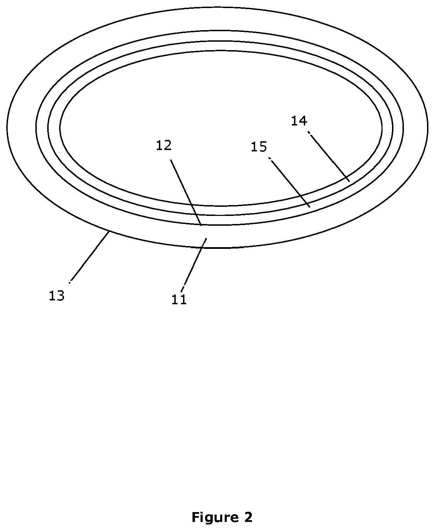

As shown in Figure 1, an elastomeric article (10), such as a glove, is provided with the additions of a therapeutic agent and an extract of soapnuts. In one aspect, the elastomeric article (10) comprises a substrate body (11) defining an inner surface (12) proximal to skin of a user and an outer surface (13) distal to the skin of the user, the substrate body (11) comprises an elastomer; an intermediate coating (15) overlying at least a portion of the inner surface (12), the intermediate coating (15) comprises a therapeutic agent; and an inner coating (14) overlying at least a portion of the intermediate coating (15), wherein the inner coating (14) comprises an extract of soapnuts for preserving the therapeutic agent. In an example where the elastomeric article (10) is a glove, the inner surface (12) forms a cavity for receiving a hand of the user and the outer surface (13) will be in contact with an external object. Figure 2 illustrates a cross sectional view of the elastomeric article (10) where the therapeutic agent and the soapnuts extract are applied sequentially to the substrate body (11), thereby forming an intermediate coating (15) of therapeutic agent and an inner coating (14) of soapnuts extract.

In another aspect, the substrate body (11) comprises an elastomer and a therapeutic agent; and an inner coating (14) overlying at least a portion of the inner surface (12), wherein the inner coating (14) comprises an extract of soapnuts for preserving the therapeutic agent.

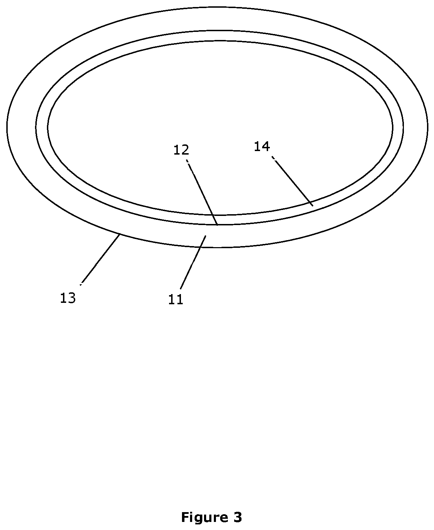

Figure 3 illustrates a cross sectional view of the elastomeric article (10) where a therapeutic agent is admixed into a substrate body (11), and an inner coating (14) of soapnuts extract is applied directly to the substrate body (11).

The therapeutic agent can therefore be incorporated into the substrate body (11) or applied to the substrate body (11) as an intermediate coating (15).

Therapeutic agents, particularly plant-based extracts are susceptible to microbial degradation. The soapnuts extract is thus provided to protect against the degradation of therapeutic agents, thereby prolong the shelf life of the articles (10).

In an alternative embodiment, the coatings of the therapeutic agents or the soapnuts extract are applied in a substantial thickness to form layers overlying the inner surface (12) of the substrate body (11).

Preferably, the article (10) comprises a preservative incorporated in the substrate body (11), the intermediate coating (15), or the inner coating (14).

More preferably, the preservative is sodium benzoate or potassium benzoate or a combination thereof. It would be appreciated by a person skilled in the art that any preservatives suitable for making the elastomeric article (10) may also be used.

The articles (10) may also include any other additives known in the art, for examples, colorants, fragrances, flavors, lubricants, or any other substances that do not adversely affect the safety and effectiveness of the articles (10).

In an embodiment, the intermediate coating (15) may comprise a combination of the plant-based extracts selected from *Aloe vera,* ginseng, and lotus. In a further embodiment, the plant-based extracts may be incorporated as multiple separate intermediate coatings (15) in any sequence. In yet a further embodiment, the intermediate coating (15) comprises a first intermediate coating incorporated with the *Aloe vera* extract, and a second intermediate coating incorporated with the ginseng extract.

Preferably, the intermediate coating (15) comprises 25-40% by volume of 1% (w/v) *Aloe vera* extract, 25-40% by volume of 1% (w/v) ginseng extract. More preferably, the intermediate coating (15) comprises 25-35% by volume of 1% (w/v) *Aloe vera* extract, 25-35% by volume of 1% (w/v) ginseng extract, 10-15% by volume of sodium benzoate, 10-15% by volume of potassium benzoate, and 0-30% by volume of water.

Preferably, the inner coating (14) comprises 20-30% by volume of 1% soapnuts extract. More preferably, the inner coating (14) comprises 20-30% by volume of 1% soapnuts extract, 4-6% by volume of sodium benzoate, 4-6% by volume of potassium benzoate, and 58-72% by volume of water.

The amount of the preservative is proportional to the amount of the therapeutic agent, that is, the higher the amount of *Aloe vera* extract and soapnuts extract, the higher the amount of sodium benzoate and potassium benzoate.

The substrate body (11) may be composed of an elastomer selected from polyurethane, acrylonitrile rubber, polychloroprene, synthetic polyisoprene, nitrile, carboxylated acrylonitrile butadiene rubber, styrene isoprene styrene, styrene ethylene butadiene styrene, butadiene co-polymer, polyvinyl chloride, natural rubber, or a combination thereof. Advantageously, the substrate body (11) of a glove is made from natural latex or nitrile.

The elastomeric articles (10) as mentioned above may include any flexible articles known in the art, for examples, gloves, liners, finger cots, sheaths, cervical caps, dental dams, or condoms.

According to another aspect of the present invention, there is provided a method of making the elastomeric article (10), comprising the steps of dipping a former of the article (10) into a dipping mixture comprising the elastomer; coating the article (10) with the therapeutic agent; and coating the article (10) with the extract of soapnuts; wherein the coating steps are performed at a low temperature.

In a further embodiment, the step of coating the article (10) with the therapeutic agent may comprise coating the article (10) with the *Aloe vera* extract and coating the article (10) with the ginseng extract.

In a further aspect of the present invention, there is provided another method of making the elastomeric article (10), comprising the steps of dipping a former of the article (10) into a dipping mixture comprising the elastomer and the therapeutic agent; and coating the article (10) with the extract of soapnuts at a low temperature.

Preferably, the step of coating the article (10) with the therapeutic agent is performed at a cold temperature, for example, at a temperature of 12°C to 15°C.

More preferably, the step of coating the article (10) with the extract of soapnuts is performed at a cool temperature, for example at a temperature of 20°C. Advantageously, the step of coating the article (10) with the extract of soapnuts is performed by spraying the article (10) with the extract of soapnuts in an enclosed space. An example of the enclosed space is a cool room.

Therapeutic agents often contain substances which are unstable under elevated temperature or heat. The existing methods require typically a temperature of above 35°C for applying the therapeutic agents, for example *Aloe vera* extract, to the substrate body (11). Considering the efficacy of the therapeutic agents can be compromised by heat, the therapeutics agents and the soapnuts extract are coated at low temperatures to maintain the efficacy and stability of the therapeutic agents. The extracts of *Aloe vera,* ginseng and soapnuts can crosslink well with the elastomer and integrate well with each other to form a stabilized product with high efficacy.

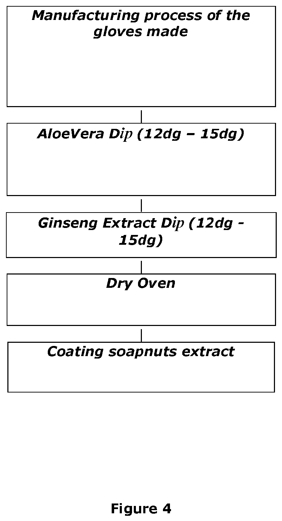

Figure 4 is an exemplary method of making a glove according to an embodiment of the present invention. The glove is made by dipping a former of the glove into a dipping mixture comprising the elastomer. The glove is then cured and prepared for the coating steps. The glove is dipped into the *Aloe vera* extract and then dipped into the ginseng extract at a temperature of 12°C to 15°C. The coated glove is dried in an oven. After drying, the glove is sprayed with the soapnuts extract in a cool room to form a fastdrying soapnuts coating. The finished glove is then stripped from the former, turning the glove inside out, and the soapnuts coating will be in contact with the skin of the user when worn.

### Examples

The following examples further illustrates the advantages of the invention and should not be construed as limiting the invention to the embodiments depicted therein.

Table 1 and Table 2 show examples of the ingredients and the volume of the ingredients for preparing the intermediate coating (15) and the inner coating (14) respectively.

**Table 1: Formulation of intermediate coating**

| Ingredient | Volume (mL) | Percentage by volume (%) |
|---|---|---|
| Aloe vera 1% (w/v) | 100 | 35.71% |
| Ginseng 1% (w/v) | 100 | 35.71% |
| Potassium sorbate | 40 | 14.29% |
| Sodium Benzoate | 40 | 14.29% |
| Total | 280 | 100.00% |

**Table 2: Formulation of inner coating**

| Ingredient | Volume (mL) | Percentage by volume (%) |
|---|---|---|
| Soapnuts 1% (w/v) | 29.7 | 26.55% |
| Potassium sorbate | 5.94 | 5.31% |
| Sodium Benzoate | 5.94 | 5.31% |
| Water | 70.3 | 62.84% |
| Total | 111.88 | 100.00% |

### Single Patch Test

A 24-hour patch test was conducted at Eurofins to assess the acute cutaneous tolerance of the gloves on 33 adult subjects. The tested gloves include gloves with the addition of an extract of *Aloe vera,* ginseng, or soapnuts, or a combination of the three extracts. The results of the clinical safety test indicated that all the tested gloves are non-irritating.

### Tensile Strength and Morphological Studies

The coating to the substrate body (11) was evaluated using two glove samples, one coated with the lotus extract and one coated with the ginseng extract. The results shows that the physical properties of the glove with the coatings are comparable to those of the control glove (with no coatings), suggesting that the coatings do not affect the physical strength of the gloves.

The morphology of the coating on the glove after one year of production was studied using Atomic Force Microscopy (AFM) imaging. Flake-like particles were observed in the images, suggesting the presence of active coating materials on the glove.

While the preferred embodiments of the present invention have been described and illustrated, it should now be apparent to those skilled in the art that various changes and modifications can be made without departing from the scope of the claims.

## Claims

1. An elastomeric article (10) comprising:
a substrate body (11) defining an inner surface (12) proximal to skin of a user and an outer surface (13) distal to the skin of the user, the substrate body (11) comprises an elastomer;
an intermediate coating (15) overlying at least a portion of the inner surface (12), the intermediate coating (15) comprises a therapeutic agent; and
an inner coating (14) overlying at least a portion of the intermediate coating (15), wherein the inner coating (14) comprises an extract of soapnuts for preserving the therapeutic agent.

2. An elastomeric article (10) comprising:
a substrate body (11) defining an inner surface (12) proximal to skin of a user and an outer surface (13) distal to the skin of the user, the substrate body (11) comprises an elastomer and a therapeutic agent; and
an inner coating (14) overlying at least a portion of the inner surface (12), wherein the inner coating (14) comprises an extract of soapnuts for preserving the therapeutic agent.

3. The elastomeric article (10) according to claim 1 or 2, wherein the therapeutic agent comprises a plant-based extract.

4. The elastomeric article (10) according to claim 3, wherein the plant-based extract is an extract of a plant selected from *Aloe vera,* ginseng, lotus, or any combination thereof.

5. The elastomeric article (10) according to claim 3, wherein the plant-based extract comprises extracts of *Aloe vera* and ginseng.

6. The elastomeric article (10) according to claim 1, wherein the article (10) comprises a preservative incorporated in the substrate body (11), the intermediate coating (15), or the inner coating (14), preferably wherein the preservative is sodium benzoate or potassium benzoate or a combination thereof.

7. The elastomeric article (10) according to claim 2, wherein the article (10) comprises a preservative incorporated in the substrate body (11) or the inner coating (14), preferably wherein the preservative is sodium benzoate or potassium benzoate or a combination thereof.

8. The elastomeric article (10) according to claims 1 and 5, wherein the intermediate coating (15) comprises a first intermediate coating incorporated with *Aloe vera* extract, and a second intermediate coating incorporated with ginseng extract.

9. The elastomeric article (10) according to claims 1 and 5, wherein the intermediate coating (15) comprises 25-40% by volume of 1% (w/v) *Aloe vera* extract, 25-40% by volume of 1% (w/v) ginseng extract.

10. The elastomeric article (10) according to claim 1 or 2, wherein the inner coating (14) comprises 20-30% by volume of 1% (w/v) soapnuts extract.

11. The elastomeric article (10) according to claim 1 or 2, wherein the elastomer is selected from polyurethane, acrylonitrile rubber, polychloroprene, synthetic polyisoprene, nitrile, carboxylated acrylonitrile butadiene rubber, styrene isoprene styrene, styrene ethylene butadiene styrene, butadiene co-polymer, polyvinyl chloride, natural rubber, or a combination thereof.

12. The elastomeric article (10) according to claim 1 or 2, wherein the article (10) is a glove, a liner, a finger cot, a sheath, a cervical cap, a dental dam, or a condom.

13. A method of making the elastomeric article (10) of claim 1, comprising the steps of:
dipping a former of the article (10) into a dipping mixture comprising the elastomer;
coating the article (10) with the therapeutic agent; and
coating the article (10) with the extract of soapnuts;
wherein the coating steps are performed at a temperature sufficiently low to preserve the efficacy of the therapeutic agent.

14. A method of making the elastomeric article (10) of claim 2, comprising the steps of:
dipping a former of the article (10) into a dipping mixture comprising the elastomer and the therapeutic agent; and
coating the article (10) with the extract of soapnuts at a temperature sufficiently low to preserve the efficacy of the therapeutic agents.

15. The method according to claim 13, wherein the step of coating the article (10) with the therapeutic agent comprises:
coating the article (10) with the *Aloe vera* extract, and
coating the article (10) with the ginseng extract.

## Patentansprüche

1. Elastomerischer Artikel (10), umfassend:
einen Substratkörper (11), der eine Innenfläche (12) proximal zu der Haut eines Benutzers und eine Außenfläche (13) distal zu der Haut des Benutzers definiert, wobei der Substratkörper (11) ein Elastomer umfasst;
eine Zwischenbeschichtung (15), die mindestens einen Teil der inneren Oberfläche (12) überlagert, wobei die Zwischenbeschichtung (15) ein therapeutisches Mittel umfasst; und
eine innere Beschichtung (14), die mindestens einen Teil der Zwischenbeschichtung (15) überlagert, wobei die innere Beschichtung (14) einen Waschnussextrakt zum Beibehalten des therapeutischen Mittels umfasst.

2. Elastomerischer Artikel (10), umfassend:
einen Substratkörper (11), der eine Innenfläche (12) proximal zu der Haut eines Benutzers und eine Außenfläche (13) distal zu der Haut des Benutzers definiert, wobei der Substratkörper (11) ein Elastomer und ein therapeutisches Mittel umfasst; und
eine innere Beschichtung (14), die mindestens einen Teil der inneren Oberfläche (12) überlagert, wobei die innere Beschichtung (14) einen Waschnussextrakt zum Beibehalten des therapeutischen Mittels umfasst.

3. Elastomerischer Artikel (10) nach Anspruch 1 oder 2, wobei das therapeutische Mittel einen pflanzenbasierten Extrakt umfasst.

4. Elastomerischer Artikel (10) nach Anspruch 3, wobei der Extrakt auf Pflanzenbasis ein Extrakt einer Pflanze ist, die ausgewählt ist aus *Aloe vera,* Ginseng, Lotus oder einer beliebigen Kombination davon.

5. Elastomerischer Artikel (10) nach Anspruch 3, wobei der pflanzliche Extrakt Extrakte von *Aloe vera* und Ginseng umfasst.

6. Elastomerischer Artikel (10) nach Anspruch 1, wobei der Artikel (10) ein Konservierungsmittel umfasst, das in den Substratkörper (11), die Zwischenbeschichtung (15) oder die Innenbeschichtung (14) integriert ist, vorzugsweise wobei das Konservierungsmittel Natriumbenzoat oder Kaliumbenzoat oder eine Kombination davon ist.

7. Elastomerischer Artikel (10) nach Anspruch 2, wobei der Artikel (10) ein Konservierungsmittel umfasst, das in den Substratkörper (11) oder in die innere Beschichtung (14) integriert ist, vorzugsweise wobei das Konservierungsmittel Natriumbenzoat oder Kaliumbenzoat oder eine Kombination davon ist.

8. Elastomerischer Artikel (10) nach Anspruch 1 und 5, wobei die Zwischenbeschichtung (15) eine erste Zwischenbeschichtung, in die Aloe-Vera-Extrakt integriert ist, und eine zweite Zwischenbeschichtung, in die Ginseng-Extrakt integriert ist, umfasst.

9. Elastomerischer Artikel (10) nach Anspruch 1 und 5, wobei die Zwischenbeschichtung (15) 25-40 Vol.-% von 1 % (Gew./Vol.) Aloe-Vera-Extrakt, 25-40 Vol.-% von 1 % (Gew./Vol.) Ginseng-Extrakt umfasst.

10. Elastomerischer Artikel (10) nach Anspruch 1 oder 2, wobei die innere Beschichtung (14) 20-30 Vol.-% von 1 % (Gew./Vol.) Waschnussextrakts umfasst.

11. Elastomerischer Artikel (10) nach Anspruch 1 oder 2, wobei das Elastomer ausgewählt ist aus Polyurethan, Acrylnitrilkautschuk, Polychloropren, synthetischem Polyisopren, Nitril, carboxyliertem Acrylnitril-Butadien-Kautschuk, Styrol-Isopren-Styrol, Styrol-Ethylen-Butadien-Styrol, Butadien-Copolymer, Polyvinylchlorid, Naturkautschuk oder einer Kombination davon.

12. Elastomerischer Artikel (10) nach Anspruch 1 oder 2, wobei der Artikel (10) ein Handschuh, ein Liner, ein Fingerling, eine Hülle, eine Portiokappe, ein Kofferdam oder ein Kondom ist.

13. Verfahren zum Fertigen des elastomerischen Artikels (10) nach Anspruch 1, umfassend die folgenden Schritte:
Eintauchen eines Formteils des Artikels (10) in ein Tauchgemisch, das das Elastomer umfasst;
Beschichten des Artikels (10) mit dem therapeutischen Mittel; und
Beschichten des Artikels (10) mit dem Waschnussextrakt;
wobei die Beschichtungsschritte bei einer ausreichend niedrigen Temperatur durchgeführt werden, um die Wirksamkeit des therapeutischen Mittels beizubehalten.

14. Verfahren zum Fertigen des elastomerischen Artikels (10) nach Anspruch 2, umfassend die folgenden Schritte:
Eintauchen eines Formteils des Artikels (10) in ein Tauchgemisch, umfassend das Elastomer und das therapeutische Mittel; und
Beschichten des Artikels (10) mit dem Waschnussextrakt bei einer ausreichend niedrigen Temperatur, um die Wirksamkeit der therapeutischen Mittel beizubehalten.

15. Verfahren nach Anspruch 13, wobei der Schritt des Beschichtens des Artikels (10) mit dem therapeutischen Mittel Folgendes umfasst:
Beschichten des Artikels (10) mit dem Aloe-Vera-Extrakt, und
Beschichten des Artikels (10) mit dem Ginseng-Extrakt.

## Revendications

1. Article élastomère (10) comprenant :
un corps de substrat (11) définissant une surface interne (12) proche de la peau d'un utilisateur et une surface externe (13) distale par rapport à la peau de l'utilisateur, le corps de substrat (11) comprenant un élastomère ;
un revêtement intermédiaire (15) recouvrant au moins une partie de la surface interne (12), le revêtement intermédiaire (15) comprenant un agent thérapeutique ; et
un revêtement interne (14) recouvrant au moins une partie du revêtement intermédiaire (15), dans lequel le revêtement interne (14) comprend un extrait de noix de lavage pour conserver l'agent thérapeutique.

2. Article élastomère (10) comprenant :
un corps de substrat (11) définissant une surface interne (12) proche de la peau d'un utilisateur et une surface externe (13) distale par rapport à la peau de l'utilisateur, le corps de substrat (11) comprenant un élastomère et un agent thérapeutique ; et
un revêtement interne (14) recouvrant au moins une partie de la surface interne (12), dans lequel le revêtement interne (14) comprend un extrait de noix de lavage pour conserver l'agent thérapeutique.

3. Article élastomère (10) selon la revendication 1 ou 2, dans lequel l'agent thérapeutique comprend un extrait végétal.

4. Article élastomère (10) selon la revendication 3, dans lequel l'extrait végétal est un extrait d'une plante choisie parmi l'*Aloe vera,* le ginseng, le lotus, ou une quelconque combinaison de ceux-ci.

5. Article élastomère (10) selon la revendication 3, dans lequel l'extrait végétal comprend des extraits d'*Aloe vera* et de ginseng.

6. Article élastomère (10) selon la revendication 1, dans lequel l'article (10) comprend un conservateur incorporé dans le corps de substrat (11), le revêtement intermédiaire (15) ou le revêtement interne (14), de préférence dans lequel le conservateur est le benzoate de sodium ou le benzoate de potassium ou une combinaison des deux.

7. Article élastomère (10) selon la revendication 2, dans lequel l'article (10) comprend un conservateur incorporé dans le corps de substrat (11) ou le revêtement interne (14), de préférence dans lequel le conservateur est le benzoate de sodium ou le benzoate de potassium ou une combinaison de ceux-ci.

8. Article élastomère (10) selon les revendications 1 et 5, dans lequel le revêtement intermédiaire (15) comprend un premier revêtement intermédiaire incorporé à l'extrait d'*Aloe vera,* et un second revêtement intermédiaire incorporé à l'extrait de ginseng.

9. Article élastomère (10) selon les revendications 1 et 5, dans lequel le revêtement intermédiaire (15) comprend 25 à 40 % en volume d'extrait d'*Aloe vera* à 1 % (p/v), 25 à 40 % en volume d'extrait de ginseng à 1 % (p/v).

10. Article élastomère (10) selon la revendication 1 ou 2, dans lequel le revêtement interne (14) comprend 20 à 30 % en volume d'extrait de noix de lavage à 1 % (p/v).

11. Article élastomère (10) selon la revendication 1 ou 2, dans lequel l'élastomère est choisi parmi le polyuréthane, le caoutchouc d'acrylonitrile, le polychloroprène, le polyisoprène synthétique, le nitrile, le caoutchouc d'acrylonitrile-butadiène carboxylé, le styrèneisoprène-styrène, le styrène-éthylène-butadiène-styrène, le copolymère de butadiène, le chlorure de polyvinyle, le caoutchouc naturel, ou une combinaison de ceux-ci.

12. Article élastomère (10) selon la revendication 1 ou 2, dans lequel l'article (10) est un gant, une doublure, un doigtier, une gaine, une cape cervicale, une digue dentaire ou un préservatif.

13. Procédé de fabrication de l'article élastomère (10) selon la revendication 1, comprenant les étapes de :
trempage d'un gabarit de l'article (10) dans un mélange de trempage comprenant l'élastomère ;
le revêtement de l'article (10) avec l'agent thérapeutique ; et
le revêtement de l'article (10) avec l'extrait de noix de lavage ;
dans lequel les étapes de revêtement sont réalisées à une température suffisamment basse pour préserver l'efficacité de l'agent thérapeutique.

14. Procédé de fabrication de l'article élastomère (10) selon la revendication 2, comprenant les étapes de :
trempage d'un gabarit de l'article (10) dans un mélange de trempage comprenant l'élastomère et l'agent thérapeutique ; et
le revêtement de l'article (10) avec l'extrait de noix de lavage à une température suffisamment basse pour préserver l'efficacité des agents thérapeutiques.

15. Procédé selon la revendication 13, dans lequel l'étape de revêtement de l'article (10) avec l'agent thérapeutique comprend :
le revêtement de l'article (10) avec l'extrait d'*aloe vera,* et
le revêtement de l'article (10) avec l'extrait de ginseng.
